(19) Europäisches Patentamt
European Patent Office
Office européen des brevets

(11) EP 4 253 552 A1

(12) **EUROPEAN PATENT APPLICATION**
published in accordance with Art. 153(4) EPC

(43) Date of publication:
**04.10.2023 Bulletin 2023/40**

(21) Application number: **21897508.4**

(22) Date of filing: **06.10.2021**

(51) International Patent Classification (IPC):
***C12P 7/62*** *(2022.01)* ***C08G 63/89*** *(2006.01)*
***C12P 7/42*** *(2006.01)*

(52) Cooperative Patent Classification (CPC):
**C08G 63/89; C12P 7/42; C12P 7/62**

(86) International application number:
**PCT/JP2021/036938**

(87) International publication number:
**WO 2022/113530 (02.06.2022 Gazette 2022/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.11.2020 JP 2020194506**

(71) Applicant: **Kaneka Corporation**
**Osaka-shi, Osaka 530-8288 (JP)**

(72) Inventors:
- **HIROTA, Shogo**
  **Takasago-shi, Hyogo 676-8688 (JP)**
- **NISHINAKA, Tomoya**
  **Takasago-shi, Hyogo 676-8688 (JP)**

(74) Representative: **Vossius & Partner**
**Patentanwälte Rechtsanwälte mbB**
**Siebertstraße 3**
**81675 München (DE)**

(54) **POLY(3-HYDROXYALKANOATE) PRODUCTION METHOD**

(57) It is an object to provide P3HA in which impurities (in particular, residual protein) are reduced, at a high yield, from microbial cells containing P3HA. The above object is attained by a method for producing P3HA having a weight average molecular weight of 100,000 to 700,000, the method including: (a) a step of adjusting the pH of a culture solution including microbial cells containing P3HA to 8.0 to 12.0; (b) a step of adding an enzyme to the culture solution to perform an enzyme treatment; and (c) a step of adjusting the pH of the culture solution to 10.0 to 12.0.

FIG. 1

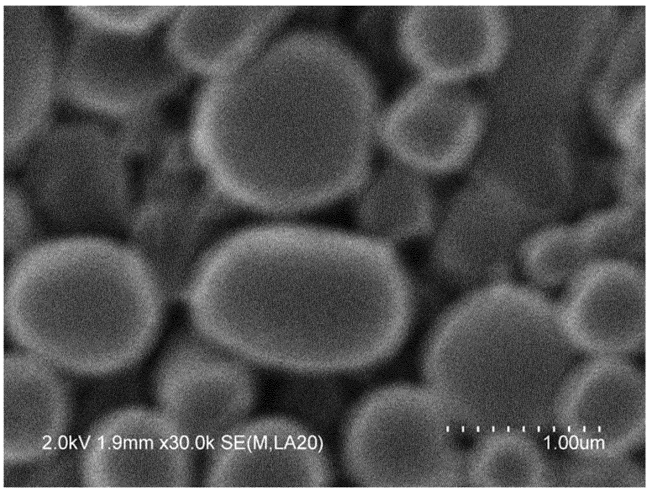


EP 4 253 552 A1

## Description

Technical Field

The present invention relates to a method for producing poly(3-hydroxyalkanoate).

Background Art

In recent years, environmental issues caused by plastic waste have been focused on. In particular, marine contamination caused by plastic waste is serious. It is therefore expected that biodegradable plastic which is degraded in a natural environment will be widespread.

Various types of such biodegradable plastic are known. Among those types of biodegradable plastic, poly(3-hydroxyalkanoate) (hereinafter may be referred to as "P3HA") is a thermoplastic polyester that is produced and accumulated as an energy storage substance in cells of many microorganism species. P3HA is a material that can undergo biodegradation not only in soil but also in seawater. Thus, P3HA has attracted attention as a material for solving the above-described problems.

For example, Patent Literature 1 describes, as a method for producing P3HA, a method of subjecting microbial cells containing P3HA to an enzyme treatment and then treating the microbial cells with an aqueous alkaline solution. In addition, Patent Literature 2 describes a method of treating microbial cells containing P3HA with an aqueous alkaline solution.

Citation List

[Patent Literature]

[Patent Literature 1]
Japanese Patent No. 2012-115145
[Patent Literature 2]
Specification of Chinese Patent Application Publication No. 111019108

Summary of Invention

Technical Problem

However, the convention techniques are excellent, but have room for further improvement.

Thus, an object of the present invention is to provide a method for producing P3HA in which impurities (in particular, residual protein) are reduced, at a high yield, from microbial cells containing P3HA.

Solution to Problem

As a result of diligent research to solve the above problems, the inventors of the present invention completed the present invention after finding for the first time that adjusting the molecular weight of P3HA by alkaline treatment of microbial cells containing P3HA before disruption of the microbial cells (before enzyme treatment) and after disruption of the microbial cells (after enzyme treatment) enabled P3HA in which impurities (in particular, residual protein) were reduced to be obtained at a high yield.

Therefore, an aspect of the present invention is a method for producing poly(3-hydroxyalkanoate) having a weight average molecular weight of 100,000 to 700,000, the method including: (a) a step of adding an aqueous alkaline solution to a culture solution including microbial cells containing poly(3-hydroxyalkanoate) to adjust the pH of the culture solution to 8.0 to 12.0; (b) a step of adding an enzyme to the culture solution obtained in the step (a) to subject the microbial cells to an enzyme treatment; and (c) a step of adding an aqueous alkaline solution to the culture solution obtained in the step (b) to adjust the pH of the culture solution to 10.0 to 12.0.

Further, an aspect of the present invention is poly(3-hydroxyalkanoate) powder having a weight average molecular weight of 100,000 to 600,000, having a residual protein content of not more than 2000 ppm, having a particle surface which is non-porous, and having a weight average molecular weight retention rate of not less than 50%, the weight average molecular weight retention rate represented by the following formula (1):

$$\text{Weight average molecular weight retention rate (\%)} = (\text{weight average molecular weight of P3HA after heating at 160°C for 20 minutes} / \text{weight average molecular weight of P3HA before heating at 160°C for 20 minutes}) \times 100 \quad \ldots (1)$$

Advantageous Effects of Invention

According to an aspect of the present invention, it is possible to provide a method for producing P3HA in which impurities (in particular, residual protein) are reduced, at a high yield, from microbial cells containing P3HA.

Brief Description of Drawings

Fig. 1 is a view showing a result of observation of the surface of P3HA powder produced in Example 1 in accordance with an embodiment of the present invention by use of an electron microscope (SEM).
Fig. 2 is a view showing a result of observation of the surface of P3HA powder produced in Comparative Example 2 by use of the electron microscope (SEM).
Fig. 3 is a diagram summarizing the operations carried out in Examples and Comparative Examples and the order of the operations.

Description of Embodiments

The following description will discuss an embodiment of the present invention in detail. Note that any numerical range expressed as "A to B" in the present specification means "not less than A and not more than B" unless otherwise stated. All literatures listed herein are incorporated herein by reference.

1. Overview of the present invention

A method for producing poly(3-hydroxyalkanoate) in accordance with an embodiment of the present invention (hereinafter referred to as "present production method") is a method for producing poly(3-hydroxyalkanoate) having a weight average molecular weight of 100,000 to 700,000, the method including: (a) a step of adding an aqueous alkaline solution to a culture solution including microbial cells containing poly(3-hydroxyalkanoate) to adjust the pH of the culture solution to 8.0 to 12.0; (b) a step of adding an enzyme to the culture solution obtained in the step (a) to subject the microbial cells to an enzyme treatment, and (c) a step of adding an aqueous alkaline solution to the culture solution obtained in the step (b) to adjust the pH of the culture solution to 10.0 to 12.0.

As a result of attempting to produce P3HA from microbial cells containing P3HA by the method described in Patent Literature 1, the inventors of the present invention found that the method described in Patent Literature 1 decreased the yield of P3HA. The inventors of the present invention found that the method described in Patent Literature 1 made the yield of P3HA extremely poor when low-molecular-weight P3HA, in particular, was produced. In addition, as a result of attempting to produce P3HA from microbial cells containing P3HA by the method described in Patent Literature 2, the inventors of the present invention found that a large amount of impurities (in particular, residual protein) were contained in P3HA obtained by the method described in Patent Literature 2. Processing with use of such P3HA generates foreign matter in a resulting product, resulting in a problem of poor quality.

Thus, as a result of diligent studies to solve the above problems, the inventors of the present invention found for the first time that adjusting the molecular weight of P3HA by alkaline treatment of microbial cells before disruption of the microbial cells (before enzyme treatment) and after disruption of the microbial cells (after enzyme treatment) enabled P3HA in which impurities (in particular, residual protein) were reduced to be obtained at a high yield. Note that, as used herein, "after disruption of microbial cells" need only be after an enzyme treatment step, which is a step (b) (described later), and means that a time during disruption of microbial cells is also included.

In addition, the inventors of the present invention found for the first time that P3HA obtained by the above-described production method has the following properties:

- Having excellent thermal stability;
- Containing a large amount of P3HA with a similar molecular weight from the viewpoint of molecular weight distribution (monodispersity); and
- In a case where low-molecular-weight P3HA is produced, the surface of obtained P3HA powder is non-porous.

As described above, there has been no report on the method for producing, at a high yield, P3HA in which impurities (in particular, residual protein) are reduced, and the production method found by the inventors of the present invention is extremely useful. In addition, conventionally, alkaline treatment was carried out only once at the production of P3HA. This is because there was a risk that, if the molecular weight of P3HA is excessively reduced by carrying out alkaline treatment multiple times, desired physical properties cannot be obtained. Under these circumstances, it is surprising that a high yield of P3HA and a small amount of low impurities (low protein) were simultaneously achieved by carrying out alkaline treatment multiple times.

Note that the inventors of the present invention presume that the following factor contributes to the obtainment of a high yield of P3HA by the present production method. That is, in the conventional method (for example, the method described in Patent Literature 1), the molecular weight adjustment is carried out after the P3HA has become crystallized. Thus, the molecular weight of the crystallized portion of P3HA cannot be sufficiently adjusted. As a result, only a terminal portion of P3HA is hydrolyzed, and small molecules which cannot be collected are generated. In contrast, in the present production method, the molecular weight adjustment is carried out before disruption of the microbial cells, so that amorphous P3HA is randomly hydrolyzed. As a result, the generation of small molecules which cannot be collected is prevented or reduced, and a high yield can be achieved.

Further, the inventors of the present invention presume that the following factor contributes to the obtainment of P3HA in which impurities (in particular, residual protein) are reduced by the present production method. That is, in the method described in the conventional method (for example, Patent Literature 2), microbial cells are cleaned with a surfactant immediately after disruption of the microbial cells. Thus, residual protein derived from the microbial cells are not sufficiently removed. In contrast, in the present production method, alkaline treatment is carried out after disruption of the microbial cells, so that impurities such as proteins derived from the microbial cells are dispersed and dissolved. Thus, it is possible to produce P3HA with few impurities.

Furthermore, the configuration described above makes it possible to prevent or reduce marine contamination caused by plastic waste. This enables the present invention to contribute to achievement of sustainable development goals (SDGs) such as Goal 12 "Ensure sustainable consumption and production patterns" and Goal 14 "Conserve and sustainably use the oceans, seas and marine resources for sustainable development".

[2. Method for producing P3HA]

The present production method is a method for producing P3HA (poly(3-hydroxyalkanoate)) having a weight average molecular weight of 100,000 to 700,000, the method including the following steps (a) to (c).

In an embodiment of the present invention, the present production method may appropriately include, for example, steps (a'), (c'), (d), (e), and (f) which will be presented below.

As used herein, the term "molecular weight" means "weight average molecular weight" unless otherwise specified. As used herein, the term "low-molecular-weight P3HA" means P3HA having a weight average molecular weight of not more than 300,000. A P3HA produced by the present production method is preferably a low-molecular-weight P3HA.

<Step (a)>

The step (a) is a step of adding an aqueous alkaline solution to a culture solution including microbial cells containing P3HA (also referred to as "alkaline treatment") to adjust the pH of the culture solution to 8.0 to 12.0. That is, it can be said that the step (a) is a step of hydrolyzing P3HA by adjustment of the pH to 8.0 to 12.0 to adjust the molecular weight of P3HA. The step (a) enables the microbial cells containing P3HA to undergo adjustment of the molecular weight of the P3HA in the microbial cells (in other words, in the state in which the P3HA is uncrystallized (amorphous)). It can also be said that the step (a) is a step of maintaining the culture solution including the microbial cells containing P3HA at a pH of 8.0 to 12.0 for a certain period of time. It can also be said that the step (a) is a molecular weight adjustment step.

(P3HA)

As used herein, the term "P3HA" means a generic term for copolymers each consisting of at least one unit represented by the following formula (2):

$$[\text{-O-CHR-CH}_2\text{-CO-}] \qquad (2)$$

(wherein R is an alkyl group represented by $C_nH_{2n+i}$, wherein n is an integer of 1 to 15).

The P3HA is not particularly limited, provided that the P3HA is included in the above formula (2).

In an embodiment of the present invention, the P3HA can be poly(3-hydroxybutyrate) in which 3-hydroxybutyrate is the only repeating unit or can be a copolymer of 3-hydroxybutyrate and another hydroxyalkanoate.

In an embodiment of the present invention, the P3HA can be a mixture of a homopolymer and one or more types of copolymers or can be a mixture of two or more types of copolymers. The form of copolymerization is not particularly limited, and can be random copolymerization, alternating copolymerization, block copolymerization, graft copolymerization, or the like.

In an embodiment of the present invention, examples of the P3HA include poly(3-hydroxybutyrate) (P3HB), poly(3-hydroxybutyrate-co-3-hydroxypropionate) (P3HB3HP), poly(3-hydroxybutyrate-co-3-hydroxyhexanoate) (P3HB3HH), poly(3-hydroxybutyrate-co-3-hydroxyvalerate) (P3HB3HV), poly(3-hydroxybutyrate-co-4-hydroxybutyrate) (P3HB4HB), poly(3-hydroxybutyrate-co-3-hydroxyoctanoate) (P3HB3HO), poly(3-hydroxybutyrate-co-3-hydroxyoctadecanoate) (P3HB3HOD), poly(3-hydroxybutyrate-co-3-hydroxydecanoate) (P3HB3HD), and poly(3-hydroxybutyrate-co-3-hydroxyvalerate-co-3-hydroxyh exanoate) (P3HB3HV3HH). From the viewpoint of achieving adjustment to a low melting point and a large processing width, P3HB3HH, P3HB4HB, and P3HB3IIP are preferable. Among these examples, P3HB and P3HB3HV are preferable, and P3HB3HH and P3HB4HB are particularly preferable, because they are easy to industrially produce.

In an embodiment of the present invention, a composition ratio of copolymer components of the P3HA is such that (3-hydroxybutyrate) / (3-hydroxyhexanoate) is preferably 99.9/0.1 (mol/mol) to 80.0/20.0 (mol/mol), more preferably 99.9/0.1 (mol/mol) to 83.0/17.0 (mol/mol), and further preferably 99.9/0.1 (mol/mol) to 85.0/15.0 (mol/mol). In a case where the compositional ratio of the copolymer components of the P3HA is within the above range, it is possible to provide P3HA having excellent processability.

In an embodiment of the present invention, the weight-average molecular weight of the P3HA before molecular weight adjustment (i.e., before addition of an aqueous alkaline solution) (hereinafter also referred to as "initial molecular weight") is not particularly limited, and is, for example, not more than 3,000,000, preferably not more than 2,800,000, and more preferably not more than 2,500,000. In a case where the initial molecular weight of the P3HA is not more than 3,000,000, there is an advantage that the molecular weight adjustment can be carried out in a relatively short period of time, resulting in excellent productivity. The lower limit of the initial molecular weight of the P3HA is not particularly limited, and can be, for example, not less than 400,000. The initial molecular weight of the P3HA is measured by the method described in Examples.

(Microbial cells containing P3HA)

As used herein, "microbial cells containing P3HA" means a microorganism that produces P3HA. That is, in an embodiment of the present invention, the P3HA is produced by a microorganism. A microorganism used in the present production method is not particularly limited, provided that the microorganism is capable of producing the P3HA within a cell of the microorganism. For example, it is possible to use a microorganism isolated from nature, a microorganism deposited at a depositary institution (for example, IFO, ATCC, or the like) for strains, or a mutant, a transformant, or the like that can be prepared from any of those microorganisms. More specific examples of the microorganism include bacteria of the genera *Cupriavidus, Alcaligenes, Ralstonia, Pseudomonas, Bacillus, Azotobacter, Nocardia,* and *Aeromonas.* Among these examples, the microorganism is preferably a microorganism belonging to the genus *Aeromonas, Alcaligenes, Ralstonia,* or *Cupriavidus.* In particular, the microorganism is more preferably a strain of *A. lipolytica, A. latus, A. caviae, A. hydrophila, C. necator,* or the like, and most preferably *C. necator.*

In a case where the microorganism is one that is inherently not capable of producing P3HA or one that produces only a small amount of P3HA, a transformant obtained by introducing, into the microorganism, a gene of an enzyme that synthesizes intended P3HA and/or a variant of the gene can be also used. The gene of such a P3HA synthetase used to prepare the transformant is not particularly limited, but is preferably a gene of a P3HA synthetase derived from *A. caviae.* By culturing these microorganisms under appropriate conditions, it is possible to obtain cells of the microorganisms having P3HAs accumulated within the cells. A method of culturing a cell of the microorganism is not particularly limited, and can be a method described in, for example, Japanese Patent Application Publication Tokukaihei No. 05-93049.

In the step (a), the microbial cells containing P3HA are preferably inactivated. An inactivation method is not particularly limited, and examples thereof include a method of subjecting the culture solution including the microbial cells containing P3HA to heating and stirring at 70°C to 80°C for 8 hours.

(Aqueous alkaline solution)

In an embodiment of the present invention, the aqueous alkaline solution is an aqueous solution that contains a basic compound. The basic compound contained in the aqueous alkaline solution is not particularly limited, and examples thereof include: hydroxides of alkali metals or alkaline earth metals such as sodium hydroxide and potassium hydroxide; metal carbonates such as sodium carbonate and potassium carbonate; and metal phosphates or metal hydrogen phosphates such as sodium phosphate, potassium phosphate, sodium hydrogen phosphate, and potassium hydrogen phosphate.

In an embodiment of the present invention, the basic compound contained in the aqueous alkaline solution is preferably an alkali metal hydroxide or an alkali earth metal hydroxide, and more preferably sodium hydroxide. One of these basic compounds may be used alone, or two or more of these basic compounds may be used in combination.

In the step (a), the aqueous alkaline solution is added to adjust the pH to preferably 8.0 to 12.0, more preferably 8.2 to 11.5, and even more preferably 8.4 to 11.5. Adjusting the pH to not less than 8.0 and maintaining the pH at a constant value (constant value of pH 8.0 to pH 12.0) makes it possible to suitably adjust the molecular weight of P3HA in the microbial cells. In addition, adjusting the pH to not more than 12.0 makes it possible to prevent unintended damage to the microbial cells. A reaction time in the step (a) (in other words, a time for adjusting and maintaining the pH at 8.0 to 12.0) is, for example, 4 to 30 hours, and preferably 8 to 20 hours. In a case where the reaction time in the step (a) is within the above range, it is possible to adjust the molecular weight of P3HA in the microbial cells to a suitable range, and it is possible to prevent the molecular weight of P3HA from being excessively reduced.

The temperature in the step (a) is preferably lower than 100°C, and more preferably lower than 80°C. A lower limit of the temperature in the step (a) is not particularly limited, and is, for example, preferably not lower than 50°C.

In the present production method, the yield of P3HA is calculated based on the following formulas (3) and (4):

P3HA content (g/g) = (weight of dry matter (g)/weight of culture solution (g)) ... (3)

Yield of P3HA (%) = (P3HA content of culture solution after molecular weight adjustment/P3HA content of culture solution before molecular weight adjustment) × 100 × (solid concentration (%) in culture solution before molecular weight adjustment/solid concentration (%) in culture solution after molecular weight adjustment) ... (4)

Various parameters in the above formulas (3) and (4) are measured by the methods described in Examples.

The present production method includes the molecular weight adjustment step (alkaline treatment step) at least twice. The "P3HA content of culture solution before molecular weight adjustment" in formula (4) means a P3HA content of a culture solution before first molecular weight adjustment is carried out, and the "P3HA content of culture solution after molecular weight adjustment" means a P3HA content of the culture solution after the final molecular weight adjustment has been carried out. Note that, as used herein, the "P3HA content of culture solution before molecular weight adjustment" and "P3HA content of culture solution after molecular weight adjustment" are values measured by the methods described in Examples.

In an embodiment of the present invention, the yield of P3HA is not lower than 95%, and is preferably not lower than 95.3% and more preferably not lower than 95.5%, from the viewpoint of productivity. The higher the yield of P3HA is, the better it is. An upper limit of the yield of P3HA is not particularly limited, and is, for example, 99%, preferably 99.5%, and most preferably 100%.

<Step (b)>

The step (b) is a step of adding an enzyme to the culture solution obtained in the step (a) to subject the microbial cells to an enzyme treatment. According to the step (b), impurities (for example, cell walls and protein) derived from the microbial cells are destroyed and removed, so that P3HA can be efficiently collected from the microbial cells.
In an embodiment of the present invention, the enzyme treatment in the step (b) can be an alkaline proteolytic enzyme treatment and/or a lytic enzyme treatment. The alkaline proteolytic enzyme treatment and the lytic enzyme treatment are preferably carried out at least once each, and if necessary, the alkaline proteolytic enzyme treatment and/or the lytic enzyme treatment may be carried out twice or more.
In an embodiment of the present invention, the order of carrying out the alkaline proteolytic enzyme treatment and/or the lytic enzyme treatment is not particularly limited.
In an embodiment of the present invention, the enzyme treatment in the step (b) is preferably such that the alkaline proteolytic enzyme treatment, the lytic enzyme treatment, and another alkaline proteolytic enzyme treatment are carried out in this order. In a case where the enzyme treatment in the step (b) is carried out in the above order, the present production method eliminates the need to use sulfuric acid or the like for adjusting the pH of the culture solution. That is, it is possible to produce P3HA without using sulfuric acid or the like, which is a liquid corrosive to an apparatus. The inventors of the present invention presume that the above order is preferable for the reason that the initial alkaline proteolytic enzyme treatment lowers the pH of the culture solution to near neutrality, resulting in the elimination of the need to add sulfuric acid or the like for pH adjustment.
In carrying out an enzyme treatment (for example, an alkaline proteolytic enzyme treatment and a lytic enzyme treatment) in the step (b), it is preferable that the pH and temperature of the culture solution be adjusted in accordance with the optimum pH and optimum temperature of an enzyme to be used. Methods for adjusting the pH and temperature of the culture solution are not particularly limited, and known methods can be used.

(Alkaline proteolytic enzyme treatment)

As used herein, the term "alkaline proteolytic enzyme" means a proteolytic enzyme having the activity of degrading protein in an alkaline environment (for example, in a solution that is pH 8.5).
In addition, as used herein, the term "alkaline proteolytic enzyme treatment" means a step of adding the alkaline proteolytic enzyme to the culture solution to subject the microbial cells to an enzyme treatment.
In an embodiment of the present invention, the alkaline proteolytic enzyme is not particularly limited, provided that the alkaline proteolytic enzyme has the activity of degrading protein in an alkaline environment. Examples of the alkaline proteolytic enzyme include serine-specific proteolytic enzymes (for example, subtilisin, chymotrypsin, and trypsin), cysteine-specific proteolytic enzymes (for example, papain, bromelain, and cathepsin), and aspartic acid-specific proteolytic enzymes (for example, pepsin, cathepsin D, and HIV protease). From the viewpoint of being economically advantageous, serine-specific proteolytic enzymes, and subtilisin (for example, alcalase), in particular, are preferable. One of these serine-specific proteolytic enzymes may be used alone, or two or more of these serine-specific proteolytic enzymes may be used in combination.
As the alkaline proteolytic enzyme, a commercially available alkaline proteolytic enzyme can be used. Examples of the commercially available alkaline proteolytic enzyme include: "Alcalase 2.5L" manufactured by Novozyme; "Protin SD-AY10" and "Protease P (Amano) 3SD" manufactured by Amano Enzyme Inc.; "Multifect PR6L" and "Optimase PR89L" manufactured by Danisco Japan Ltd.; "Sumizyme MP" manufactured by Shin Nihon Chemical Co., Ltd.; "Delvolase" manufactured by DSM Japan K.K.; "Biopurase OP", "Biopurase SP-20FG", and "Biopurase SP-4FG" manufactured by Nagase ChemteX Corporation; "Orientase 22BF" manufactured by HBI Enzymes Inc.; and "AROASE XA-10" manufactured by Yakult Pharmaceutical Industry Co., Ltd.
In an embodiment of the present invention, the optimal pH of the alkaline proteolytic enzyme is not particularly limited, provided that the alkaline proteolytic enzyme has activity in an alkaline environment. The optimal pH of the alkaline proteolytic enzyme is, for example, 8.0 to 14.0, preferably 8.0 to 12.0, more preferably 8.0 to 10.0, even more preferably 8.0 to 9.0, and most preferably 8.5.
In an embodiment of the present invention, the optimum temperature of the alkaline proteolytic enzyme is not particularly limited, but is preferably not higher than 60°C, and more preferably not higher than 50°C, from the viewpoint of eliminating the need for excessive heating and preventing thermal change (thermal decomposition) of P3HA. A lower limit of the optimum temperature is not particularly limited, but is preferably a temperature that is not lower than room temperature (for example, 25°C), from the viewpoint of eliminating the need for excessive cooling operation and being economical.

(Lytic enzyme treatment)

In an embodiment of the present invention, the alkaline proteolytic enzyme treatment is a step of adding a lytic enzyme to the culture solution to subject the microbial cells to an enzyme treatment.
As used herein, the term "lytic enzyme" means an enzyme that has the activity of degrading (lysis) cell walls (for example, peptidoglycan) of microbial cells.
In an embodiment of the present invention, the lytic enzyme is not particularly limited, and examples thereof include lysozyme, labyrinthine, β-N-acetylglucosaminidase, endolysin, and autolysin. Lysozyme is preferable from the viewpoint of being economically advantageous. One of these lytic enzymes may be used alone, or two or more of these lytic enzymes may be used in combination.
As the lytic enzyme, a commercially available lytic enzyme can also be used. Examples of the commercially available lytic enzyme include "Lysozyme" and "Achromopeptidase" manufactured by FUJIFILM Wako Pure Chemical Corporation.
In an embodiment of the present invention, the optimal pH of the lytic enzyme is not particularly limited, provided that the lytic enzyme has cell wall degradation activity. The optimal pH of the lytic enzyme is, for example, 5.0 to 11.0, preferably 6.0 to 9.0, and more preferably 6.0 to 8.0.
In an embodiment of the present invention, the optimum temperature of the lytic enzyme is not particularly limited, but is preferably not higher than 60°C, and more preferably not higher than 50°C, from the viewpoint of eliminating the need for excessive heating and preventing thermal change (thermal decomposition) of P3HA. A lower limit of the optimum temperature is not particularly limited, but is preferably a temperature that is not lower than room temperature (for example, 25°C), from the viewpoint of eliminating the need for excessive cooling operation and being economical.
In an embodiment of the present invention, the enzyme treatment in the step (b) can be carried out with a combination of lysozyme and alcalase.
An enzyme treatment time in the step (b) may vary depending on the type of enzyme, pH, temperature, and other conditions, but is, for example, 1 to 8 hours, and preferably 2 to 6 hours.

<Step (a')>

The present production method may further include a step (a') between the steps (a) and (b).
The step (a') is a step of adjusting the pH of the culture solution obtained in the step (a) to 6.0 to 8.0, and the enzyme treatment in the step (b) includes carrying out the lytic enzyme treatment and the alkaline proteolytic enzyme treatment in this order. By performing the step (a'), the enzyme treatment in the step (b) proceeds smoothly.
In the step (a'), a method of adjusting the pH is not particularly limited. For example, the pH can be adjusted by a method of adding an acid to the culture solution. The acid is not limited to a particular one, and can be selected from an organic acid and an inorganic acid. The acid can have volatility or not have volatility. More specifically, examples of the acid include sulfuric acid, hydrochloric acid, phosphoric acid, and acetic acid.
For the lytic enzyme treatment and the alkaline proteolytic enzyme treatment which are performed after the step (a'), the description under the <Step (b)> section is incorporated by reference.

<Step (c)>

The step (c) is a step of adding an aqueous alkaline solution to the culture solution obtained in the step (b) to adjust the pH to 10.0 to 12.0. The step (c) enables the molecular weight of P3HA to be adjusted to a much lower molecular weight, and enables impurities (for example, nucleic acids and protein) derived from the microbial cells to be dispersed and dissolved when the microbial cells are disrupted, so that P3HA with high purity can be separated from the microbial cells. It can also be said that the step (c) is an alkaline cleaning step.
For the aqueous alkaline solution added in the step (c), the description under the <Step (a)> section is incorporated by reference.
In the step (c), the aqueous alkaline solution is added to adjust the pH to preferably 10.0 to 12.0, more preferably 10.2 to 11.8, and even more preferably 10.4 to 11.6. Adjusting the pH to not less than 10.0 makes it possible to adjust the molecular weight of P3HA in the microbial cells to a much lower molecular weight. In addition, adjusting the pH to not more than 12.0 makes it possible to prevent the molecular weight of P3HA from being excessively reduced.
A reaction time in the step (c) (in other words, a time for adjusting and maintaining the pH at 10.0 to 12.0) is, for example, 1 to 12 hours, and preferably 1 to 6 hours. In a case where the reaction time in the step (c) is within the above range, it is possible to adjust the molecular weight of P3HA in the microbial cells to a suitable range, and it is possible to prevent the molecular weight of P3HA from being excessively reduced.

<Step (c')>

The present production method may further include a step (c') after the step (c). It can also be said that the step (c') is a surfactant treatment step.
The step (c') is a step of adding a surfactant to the culture solution obtained in the step (c). The step (c') enables efficient treatment of impurities contained in the microbial cells, cell membranes in particular, and enables removal of more impurities derived from the microbial cells, so that P3HA with higher purity can be separated from the microbial cells.
In an embodiment of the present invention, the surfactant is not particularly limited, but examples of the surfactant include anionic surfactants, cationic surfactants, amphoteric surfactants, and nonionic surfactants. Among these surfactants, anionic surfactants are preferable from the viewpoint of having high ability to remove cell membranes. One of these surfactants may be used alone, or two or more of these surfactants may be used in combination.
Examples of the anionic surfactants include alkyl sulfates, alkylbenzene sulfonates, alkyl sulfate ester salts, alkenyl sulfate ester salts, alkyl ether sulfate ester salts, alkenyl ether sulfate ester salts, α-olefin sulfonates, α-sulfofatty acid salts, esters of α-sulfofatty acid salts, alkyl ether carboxylates, alkenyl ether carboxylates, amino acid-type surfactants, and N-acylamino acid type surfactants. Among these anionic surfactants, alkyl sulfate ester salts are preferable, and sodium dodecyl sulfate (SDS) is particularly preferable from the viewpoint of having high ability to remove cell membranes and being inexpensive. One of these anionic surfactants may be used alone, or two or more of these anionic surfactants may be used in combination.
In the step (c'), the amount of surfactant to be added is not particularly limited, and is, for example, 0. 1% by weight to 5.0% by weight, and preferably 0.3% by weight to 2.5% by weight, relative to the culture solution.
In another embodiment of the present invention, the step (c') may be carried out before the step (c) or simultaneously with the step (c). That is, the surfactant may be added before the addition of the aqueous alkaline solution to the culture solution or simultaneously with the addition of the aqueous alkaline solution. Moreover, the addition of the surfactant can be carried out before the step (a) or simultaneously with the step (a).

<Step (d)>

In an embodiment of the present invention, the present production method may further include a step (d).
The step (d) is a step of centrifuging the culture solution obtained in the step (c) and removing a supernatant to obtain an aqueous P3HA suspension in which P3HA is concentrated. That is, the step (d) is a step of removing impurities from P3HA separated from the microbial cells and concentrating and purifying the P3HA. It can also be said that the step (d) is a centrifugation step.
In the step (d), a method for centrifuging the culture solution is not particularly limited, and a known method can be used.
In the step (d), after the centrifugation of the culture solution and the removal of the supernatant, it is preferable to repeatedly carry out an operation in which a solution is added to a precipitate, another centrifugation is carried out, and a supernatant is removed. With this operation, it is possible to obtain a more concentrated and purified aqueous P3HA suspension. Here, the solution added after the removal of the supernatant is preferably an aqueous alkaline solution that has been adjusted to the same pH as that of the culture solution. In an embodiment of the present invention, the solution is preferably the same as the aqueous alkaline solution used in the step (c).
The amount of the impurities which are to remain in an end product is substantially determined by the step (d). As such, it is preferable to minimize the impurities. Of course, depending on the purpose of use, it may be acceptable to have the impurities mixed in the end product, provided that the physical properties of the end product are not impaired. However, in a case where a highly pure P3HA is required, for example, for medical use, it is preferable to minimize the impurities. In so doing, an index of a degree of refinement can be, for example, a protein content of the aqueous P3HA suspension. The protein content of the aqueous P3HA suspension is not particularly limited, provided that the protein content of the aqueous P3HA suspension is a content which enables achievement of the residual protein content of P3HA powder described later. The protein content is preferably not more than 3000 ppm, more preferably not more than 2500 ppm, even more preferably not more than 2000 ppm, per weight of the P3HA in the aqueous P3HA suspension.
A solvent (the "solvent" may also be referred to as "aqueous medium") contained in the aqueous P3HA suspension in the step (d) is not particularly limited and may be water or a mixed solvent of water and an organic solvent. In the mixed solvent, the concentration of the organic solvent is not particularly limited, provided that the concentration is equal to or lower than the solubility, in water, of the organic solvent used. The organic solvent is not particularly limited. Examples of the organic solvent include: alcohols such as methanol, ethanol, 1-propanol, 2-propanol, 1-butanol, 2-butanol, iso-butanol, pentanol, hexanol, and heptanol; ketones such as acetone and methyl ethyl ketone; ethers such as tetrahydrofuran and dioxane; nitriles such as acetonitrile and propionitrile; amides such as dimethylformamide and acetamide; dimethyl sulfoxide; pyridine; and piperidine. Among these examples, methanol, ethanol, 1-propanol, 2-propanol, 1-butanol, 2-butanol, iso-butanol, acetone, methyl ethyl ketone, tetrahydrofuran, dioxane, acetonitrile, propionitrile,

and the like are preferable, because they are easy to remove. Further, methanol, ethanol, 1-propanol, 2-propanol, butanol, acetone, and the like are more preferable, because they are easy to obtain. Furthermore, methanol, ethanol, and acetone are particularly preferable.

The amount of water contained in the aqueous medium which is contained in the aqueous P3HA suspension is preferably not less than 5% by weight, more preferably not less than 10% by weight, even more preferably not less than 30% by weight, and particularly preferably not less than 50% by weight.

Note that the aqueous P3HA suspension in the step (d) may contain another solvent, a microbial cell-derived component, a compound which is generated during refinement, and/or the like, provided that the essentials of the present invention are not impaired.

<Step (e)>

In an embodiment of the present invention, the present production method can further include a step (e).

The step (e) is a step of adding a dispersing agent to the aqueous P3HA suspension obtained in the step (d) and preparing an aqueous P3HA suspension having a pH of not more than 7. It can be said that the step (e) is a pH adjustment step.

The step (e) preferably includes the following steps (e1) and (e2).

- Step (e1): adding a dispersing agent to an aqueous P3HA suspension
- Step (e2): adjusting a pH of the aqueous P3HA suspension to not more than 7

The order of carrying out the step (e1) and the step (e2) is not particularly limited, but it is preferable to carry out the step (e2) after the step (e1), from the viewpoint of reducing or preventing agglomeration of P3HA in the step (e2) and obtaining an aqueous suspension that is even more excellent in dispersion stability of P3HA.

The dispersing agent to be added in the step (e) is not particularly limited, and examples thereof include polyvinyl alcohol (PVA)-based dispersing agents, alkylene oxide-based dispersing agents, cellulose-based dispersing agents, and alcohol-based dispersing agents. Polyvinyl alcohol-based dispersing agents and alkylene oxide-based dispersing agents are preferable because they are highly effective in reducing or preventing agglomeration of P3HA in the suspensions, and alkylene oxide-based dispersing agents are more preferable because a resulting P3HA powder has high processability. One of these dispersing agents may be used alone, or two or more of these dispersing agents may be used in combination.

In an embodiment of the present invention, the alkylene oxide-based dispersing agent is not limited to a particular one, provided that it provides the above-described effect. It is preferable that the alkylene oxide-based dispersing agent be constituted by a block of poly(ethylene oxide) (PEO) and a block of poly(propylene oxide) (PPO), and be in a form of PEO-PPO-PEO.

As used herein, the term "block of poly(ethylene oxide) (PEO)" means a polymer moiety formed by polymerization of ethylene oxide (EO) in a structure of the alkylene oxide-based dispersing agent.

As used herein, the term "block of poly(propylene oxide) (PPO)" means a polymer moiety formed by polymerization of propylene oxide (PO) in the structure of the alkylene oxide-based dispersing agent.

In an embodiment of the present invention, in a case where a molecular weight of PEO in the alkylene oxide-based dispersing agent and a ratio of the molecular weight of the PEO to a molecular weight of PPO in the alkylene oxide-based dispersing agent are each within a specific range, it is possible to maintain the viscosity of the aqueous suspension and produce P3HA with high productivity.

In an embodiment of the present invention, it is preferable that a range of a molecular weight of PEO in the alkylene oxide-based dispersing agent and a range of a ratio of the molecular weight of the PEO to a molecular weight of PPO in the alkylene oxide-based dispersing agent be a combination of the following ranges.

Note that, as used herein, a "molecular weight of PEO" may be referred to as an "amount of EO", and a "molecular weight of PPO" may be referred to as an "amount of PO".

That is, in an embodiment of the present invention, a molecular weight of PEO in the alkylene oxide-based dispersing agent need only be not less than 1500, and is preferably not less than 1750 and more preferably not less than 2000. Further, in an embodiment of the present invention, an upper limit of a molecular weight of PEO in the alkylene oxide-based dispersing agent is, for example, not more than 30000, preferably not more than 25000, and more preferably not more than 20000.

In an embodiment of the present invention, a ratio of a molecular weight of PEO to a molecular weight of PPO in the alkylene oxide-based dispersing agent need only be not less than 0.5, and is preferably not less than 0.6 and more preferably not less than 0.7. An upper limit of the ratio of the molecular weight of the PEO to the molecular weight of the PPO is not more than 5.0, preferably not more than 4.8, and even more preferably not more than 4.5.

In an embodiment of the present invention, in a case where a molecular weight of PEO in the alkylene oxide-based dispersing agent and a ratio of the molecular weight of the PEO to a molecular weight of PPO in the alkylene oxide-based dispersing agent are within the above ranges, the alkylene oxide-based dispersing agent is hydrophilic, and the number of molecules increases relative to a weight of the alkylene oxide-based dispersing agent added. This makes it easier to maintain the dispersibility of the aqueous suspension.

In an embodiment of the present invention, a molecular weight of PEO in the alkylene oxide-based dispersing agent is not less than 1500, and a ratio of the molecular weight of the PEO to a molecular weight of PPO in the alkylene oxide-based dispersing agent is 0.5 to 5.0.

In an embodiment of the present invention, the alkylene oxide-based dispersing agent includes preferably at least one, more preferably at least two, PEO blocks each having a molecular weight of not less than 750. An upper limit of the number of such PEO blocks is not particularly limited, and is, for example, not more than 4, and preferably not more than 3. In a case where the number of the PEO blocks is within the above range, the alkylene oxide-based dispersing agent is hydrophilic.

In an embodiment of the present invention, a molecular weight of PPO in the alkylene oxide-based dispersing agent is not particularly limited, and is, for example, not less than 500, and preferably not less than 1500. Further, in an embodiment of the present invention, an upper limit of a molecular weight of PPO in the alkylene oxide-based dispersing agent is, for example, not more than 6700, and preferably not more than 6250. In a case where a molecular weight of PPO in the alkylene oxide-based dispersing agent is within the above range, the alkylene oxide-based dispersing agent is hydrophobic.

In an embodiment of the present invention, the number of PPO blocks in the alkylene oxide-based dispersing agent is not particularly limited, provided that the above-described effect is exhibited. The number can be one, or can be more than one (for example, 2, 3, or 4).

In an embodiment of the present invention, the alkylene oxide-based dispersing agent is, for example, a compound represented by the following formula (5):

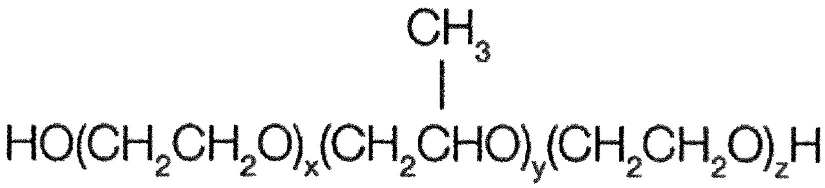

・・・(5)

wherein X is, for example, 17 to 340, preferably 20 to 285, and more preferably 22 to 226. In a case where X is not more than 340, the number of molecules increases relative to a weight of the alkylene oxide-based dispersing agent added, and thus it is easier to maintain the dispersibility of the aqueous suspension. In a case where X is not less than 17, the alkylene oxide-based dispersing agent is hydrophilic. Y is, for example, 8 to 115, preferably 10 to 110, and more preferably 24 to 107. In a case where Y is not more than 115, the alkylene oxide-based dispersing agent is dissolved in water easily. In a case where Y is not less than 8, the alkylene oxide-based dispersing agent is hydrophobic. Z is, for example, 17 to 340, preferably 20 to 285, and more preferably 22 to 226. In a case where Z is not more than 340, the number of molecules increases relative to a weight of the alkylene oxide-based dispersing agent added, and thus it is easier to maintain the dispersibility of the aqueous suspension. In a case where Z is not less than 17, the alkylene oxide-based dispersing agent is hydrophilic.

Further, in the formula (5), a sum of X and Z (may be hereinafter referred to as "X+Z") is, for example, 34 to 680, preferably 40 to 570, and more preferably 44 to 452. In a case where X+Z is not more than 680, the number of molecules increases relative to a weight of the alkylene oxide-based dispersing agent added, and thus it is easier to maintain the dispersibility of the aqueous suspension. In a case where X is not less than 34, the alkylene oxide-based dispersing agent is hydrophilic.

In an embodiment of the present invention, a commercially available alkylene oxide-based dispersing agent may be used as the alkylene oxide-based dispersing agent. Examples of the commercially available alkylene oxide-based dispersing agent include Pluronic 10400 (manufactured by BASF), Pluronic 10500 (manufactured by BASF), Genapol PF80 (manufactured by Clariant), Unilube DP60-600B (manufactured by NOF CORPORATION), Unilube DP60-950B (manufactured by NOF CORPORATION), Plonon 208 (manufactured by NOF CORPORATION), Epan U105 (manufactured by Dai-ichi Kogyo Seiyaku Co., Ltd.), Epan U108 (manufactured by Dai-ichi Kogyo Seiyaku Co., Ltd.), and Epan 750 (manufactured by Dai-ichi Kogyo Seiyaku Co., Ltd.).

An amount of the dispersing agent added to the aqueous P3HA suspension in the step (e) is not particularly limited, but is preferably 0.1 parts by weight to 20 parts by weight, more preferably 0.5 parts by weight to 10 parts by weight, and even more preferably 0.75 parts by weight to 5 parts by weight relative to 100 parts by weight of the P3HA

contained in the suspension. In a case where the amount of the dispersing agent added is within the above range, the dispersion stability of the P3HA in the aqueous P3HA suspension is further improved, so that P3HA powder can be produced efficiently.

In the step (e), the pH of the aqueous P3HA suspension after the addition of the dispersing agent is adjusted to preferably not more than 7, more preferably not more than 5, and even more preferably not more than 4, from the viewpoint of reducing coloring of P3HA when the P3HA is heated and melted and from the viewpoint of ensuring the stability of a molecular weight during heating and/or drying. Further, the pH of the aqueous P3HA suspension after the addition of the dispersing agent is adjusted to preferably not less than 1, more preferably not less than 2, and even more preferably not less than 3, from the viewpoint of acid resistance of a container. In a case where the pH of the aqueous P3HA suspension is not more than 7, coloring during heating and melting (processing) of P3HA powder is reduced, and a decrease in molecular weight during heating and/or drying is reduced or prevented.

In the step (e), a method of adjusting the pH is not particularly limited. For example, the pH can be adjusted by addition of an acid or by other methods. The acid is not limited to a particular one, and can be selected from an organic acid and an inorganic acid. The acid can have volatility or not have volatility. More specifically, examples of the acid include sulfuric acid, hydrochloric acid, phosphoric acid, and acetic acid.

A concentration of P3HA in the aqueous P3HA suspension obtained by the step (e) is preferably not less than 30% by weight, more preferably not less than 40% by weight, and even more preferably not less than 50% by weight, from the viewpoint of (i) an economical advantage in terms of utility in drying and (ii) a resultant improvement in productivity. An upper limit of the concentration of the P3HA is preferably not more than 65% by weight, more preferably not more than 60% by weight, because, otherwise, it may not be possible to ensure sufficient flowability due to closest packing. A method of adjusting the concentration of the P3HA is not particularly limited. For example, the concentration can be adjusted by addition of an aqueous medium and/or by removal of part of an aqueous medium (for example, by centrifugal separation followed by removal of a supernatant). The adjustment of the concentration of the P3HA can be carried out at any stage in the step (e), or can be carried out prior to the step (e).

<Step (f)>

In an embodiment of the present invention, the present production method can further include a step (f).

The step (f) is a step of drying the aqueous P3HA suspension prepared in the step (e). Examples of a drying method include drying under reduced pressure and a method in which the aqueous P3HA suspension in a state of fine droplets is supplied into a dryer and dried in contact with hot air in the dryer (spray-drying). Spray-drying is preferably used. It can also be said that the step (f) is a drying step.

In a case where spray-drying is carried out in the step (f), a method (atomizer) of supplying the aqueous P3HA suspension in a state of fine droplets into a dryer is not particularly limited, and can be a publicly known method such as a method using a rotary disc or a method using a nozzle. A manner of contact between the droplets and the hot air in the dryer is not particularly limited. For example, the droplets and the hot air can be brought in contact with each other in a co-current manner, a countercurrent manner, or in a manner combining a co-current manner and a countercurrent manner.

In a case where spray-drying is carried out in the step (f), a drying temperature may be any temperature at which most of the aqueous medium can be removed from the droplets of the aqueous P3HA suspension. The drying temperature can be set as appropriate, provided that the aqueous P3HA suspension can be dried until a desired moisture content is achieved and that a deterioration in quality (a decrease in molecular weight, a reduction in color tone, and the like), melting, and the like are minimized. For example, a temperature of hot air to be blown into a spray dryer can be selected as appropriate within a range of 100°C to 300°C. A volume of hot air in the dryer can be set as appropriate, for example, in accordance with a size of the dryer and the like.

In a case where spray-drying is carried out in the step (f), a step (for example, a step of subjecting P3HA to drying under reduced pressure, or the like) of further drying resultant P3HA (P3HA powder or the like) can be included after the step (f). The present production method can include other steps (for example, a step of adding various additives to the aqueous P3HA suspension, or the like).

3. P3HA powder

P3HA powder in accordance with an embodiment of the present invention (hereinafter referred to as "present P3HA powder") has a weight average molecular weight of 100,000 to 600,000, has a residual protein content of not more than 2000 ppm, has a particle surface which is non-porous, and has a weight average molecular weight retention rate of not less than 50%, the weight average molecular weight retention rate represented by the following formula (1):

$$\text{Weight average molecular weight retention rate (\%)} = \text{(weight average molecular weight of P3HA after heating at 160°C for 20 minutes/weight average molecular weight of P3HA before heating at 160°C for 20 minutes)} \times 100 \ldots (1)$$

The present P3HA powder is extremely useful in various fields because the present P3HA powder does not generate foreign matter during processing and has excellent productivity and high thermal stability.

In an embodiment of the present invention, the present P3HA powder is produced by the above-described present production method. Specifically, the present P3HA powder can be produced by, for example, a method for producing P3HA including the step (a), a method for producing P3HA including the steps (a) to (c), or the like method. Regarding P3HA, for matters other than the matters specified in this section, the description under the "2. Method for producing P3HA" section is incorporated by reference.

The weight average molecular weight of the present P3HA powder is 100,000 to 700,000, preferably 100,000 to 600,000, more preferably 100,000 to 500,000, even more preferably 100,000 to 400,000, particularly preferably 100,000 to 300,000, and most preferably 100,000 to 250,000. In a case where the weight average molecular weight of the present P3HA powder is not more than 700,000 (in particular, not more than 600,000), the use of the present P3HA powder during thermal processing enables improvements in injection moldability and mechanical properties of a molded product due to the nucleating agent effect. Low-molecular-weight P3HA powder, in particular, is preferable because the low-molecular-weight P3HA powder has more excellent nucleating agent effect. The weight average molecular weight of the present P3HA powder is denoted as "final molecular weight" in Examples. The weight average molecular weight of the present P3HA powder is measured by the method described in Examples (under the "<Final molecular weight>" section).

The ratio (Mw/Mn) of the weight average molecular weight (final molecular weight) (Mw) of the present P3HA powder to the number average molecular weight (Mn) thereof is not particularly limited, but is preferably 1.0 to 3.1, more preferably 1.2 to 3.0, and even more preferably 1.4 to 2.9, from the viewpoint of excellent molecular weight dispersibility. The Mw/Mn of the present P3HA powder is measured by the method described in Examples. Note that the number average molecular weight (Mn) is a value used for evaluation of the ratio (dispersion degree) with respect to the weight average molecular weight (final molecular weight) (Mw), and is a value that increases or decreases approximately in proportion to Mw. The fact that the Mw/Mn of the P3HA powder is 1.0 to 3.1 means that the degree of non-uniformity in molecular weight of the P3HA powder is small, in other words, means that the molecular weight of the P3HA powder is monodisperse. According to the present production method, it is possible to obtain P3HA powder having an Mw/Mn of 1.0 to 3.1.

The present P3HA powder is such that the surfaces of P3HA particles in the P3HA powder are non-porous. In a case where the surfaces of the P3HA particles are non-porous, the dispersibility is good. Thus, the particles are not agglomerated, and the alkali used in the production process does not remain inside an agglomerate. As a result, residual alkali does not act as a hydrolysis catalyst during thermal processing, resulting in good thermal stability. Whether or not the surfaces of the P3HA particles are non-porous is measured by the method described in Examples.

The present P3HA powder has a weight average molecular weight retention rate represented by the formula (1) of not less than 60%, preferably not less than 63%, more preferably not less than 65%, and particularly preferably not less than 68%. The higher the weight average molecular weight retention rate represented by the formula (1) is, the better it is, from the viewpoint of preventing deterioration during heating (during thermal processing) of the P3HA powder. In a case where the weight average molecular weight retention rate represented by the formula (1) is not less than 50%, it is possible to prevent severe deterioration at least during heating (during thermal processing). The higher the weight average molecular weight retention rate represented by the formula (1) is, the better it is. An upper limit of the weight average molecular weight retention rate represented by the formula (1) is not particularly limited, but is, for example, 90%, preferably 95%, more preferably not more than 98%, and most preferably 100%. The weight average molecular weight retention rate represented by the formula (1) is measured by the method described in Examples. Note that, when the particle surface of P3HA is porous, the weight average molecular weight retention rate represented by the formula (1) tends to decrease.

The residual protein content of the present P3HA powder is not more than 2000 ppm, preferably not more than 1900 ppm, more preferably not more than 1800 ppm. The residual protein content of the present P3HA powder can serve as an indicator of the amount of various components in the P3HA powder that have been generated or have not been removed during the production process. In a case where the residual protein content of the present P3HA is not

more than 2000 ppm, impurities are less likely to affect the physical properties of the P3HA powder, and it is possible to provide P3HA powder of stable quality that does not generate foreign matter during processing. The lower the residual protein content of the present P3HA is, the better it is, and a lower limit of the residual protein content is not particularly limited, but is, for example, not less than 200 ppm, preferably not less than 100 ppm, and more preferably not less than 50 ppm, and may be 0 ppm. The residual protein content of the present P3HA powder is measured by the method described in Examples.

A bulk density of the present P3HA powder is not particularly limited, but is preferably not less than 0.20 kg/mL, more preferably not less than 0.25 kg/mL, and even more preferably not less than 0.30 kg/mL, from the viewpoint of achieving an excellent flowability. An upper limit of the bulk density of the present P3HA powder is not particularly limited, but can be, for example, not more than 0.50 mg/mL. The bulk density of the present P3HA powder is measured by the following method. That is, in accordance with a method described in JIS K-7365, measurement is carried out using an apparatus including (i) a metal cylinder (receiver) which has a smoothened inner surface, a volume of 100 ml $\pm$ 0.5 ml, and an inner diameter of 45 mm $\pm$ 5 mm and (ii) a funnel which is set above the metal cylinder, has a lower opening of 20 mm to 30 mm, and has a damper (e.g. a metal plate) attached to the funnel. A scale capable of measuring weights in decigrams is used.

A median particle size (average particle size) of the present P3HA powder is not particularly limited, but is preferably 60 $\mu$m to 1000 $\mu$m and more preferably 100 $\mu$m to 500 pm, from the viewpoint of dust explosion prevention and powder flowability. The median particle size of the present P3HA powder is measured with use of a laser diffraction/ scatter particle size distribution meter LA-950 (manufactured by HORIBA). Specifically, to 20 ml of ion exchange water, 0.05 g of sodium dodecyl sulfate, which is a surfactant, is added as a dispersing agent to obtain an aqueous solution of the surfactant. Then, 0.2 g of P3HA powder is added to the aqueous solution of the surfactant and dispersed in the aqueous solution of the surfactant to obtain a P3HA dispersion. Thereafter, the P3HA dispersion is introduced into the laser diffraction/ scatter particle size distribution meter manufactured by HORIBA, and the median particle size of P3HA powder is measured.

The present invention is not limited to the embodiments, but can be altered by a skilled person in the art within the scope of the claims. The present invention also encompasses, in its technical scope, any embodiment derived by combining technical means disclosed in differing embodiments.

That is, an embodiment of the present invention is as follows:

<1> A method for producing poly(3-hydroxyalkanoate) having a weight average molecular weight of 100,000 to 700,000, the method including:

(a) a step of adding an aqueous alkaline solution to a culture solution including microbial cells containing poly(3-hydroxyalkanoate) to adjust the pH of the culture solution to 8.0 to 12.0;
(b) a step of adding an enzyme to the culture solution obtained in the step (a) to subject the microbial cells to an enzyme treatment; and
(c) a step of adding an aqueous alkaline solution to the culture solution obtained in the step (b) to adjust the pH of the culture solution to 10.0 to 12.0.

<2> The method described in <1>, wherein the enzyme treatment in the step (b) includes carrying out an alkaline proteolytic enzyme treatment, a lytic enzyme treatment, and another alkaline proteolytic enzyme treatment in this order.
<3> The method described in <1> or <2>, further including, between the steps (a) and (b), (a') a step of adjusting the pH of the culture solution obtained in the step (a) to 6.0 to 8.0, wherein
the enzyme treatment in the step (b) comprises carrying out a lytic enzyme treatment and an alkaline proteolytic enzyme treatment in this order.
<4> The method described in any one of <1> to <3>, wherein the enzymes in the step (b) are lysozyme and alcalase.
<5> The method described in any one of <1> to <4>, further including, after the step (c), (c') a step of adding a surfactant to the culture solution obtained in the step (c).
<6> The method described in any one of <1> to <5>, wherein a reaction time in the step (a) is 4 hours to 30 hours.
<7> The method described in any one of <1> to <6>, wherein the poly(3-hydroxyalkanoate) is poly(3-hydroxybutyrate-co-3-hydroxyhexanoate).
<8> Poly(3-hydroxyalkanoate) powder having a weight average molecular weight of 100,000 to 600,000, having a residual protein content of not more than 2000 ppm, having a particle surface which is non-porous, and having a weight average molecular weight retention rate of not less than 50%, the weight average molecular weight retention rate represented by the following formula (1):

$$\text{Weight average molecular weight retention rate (\%)} = (\text{weight average molecular weight of P3HA after heating at } 160^\circ\text{C for 20 minutes}/\text{weight average molecular weight of P3HA before heating at } 160^\circ\text{C for 20 minutes}) \times 100 \ldots (1)$$

<9> The poly(3-hydroxyalkanoate) powder described in <8>, wherein
a ratio (Mw/Mn) of a weight average molecular weight (Mw) of the poly(3-hydroxyalkanoate) powder to a number average molecular weight (Mn) thereof is 1.0 to 3.1.

## Examples

The following description will discuss embodiments of the present invention in further detail on the basis of Examples. However, the present invention is not limited by the Examples. Note that, in the Examples, "P3HB3HH" is used as "P3HA", and "P3HA" can be read as "P3HB3HH".

### Measurement method and evaluation method

In Examples and Comparative Examples, measurement and evaluation were carried out by the following methods.

<Thermal stability>

Thermal stability was evaluated as the weight average molecular weight retention rate of P3HA after heating at 160°C for 20 minutes. The weight average molecular weight retention rate of P3HA was calculated by the following formula (1):

$$\text{Weight average molecular weight retention rate (\%)} = (\text{weight average molecular weight of P3HA after heating}/\text{weight average molecular weight of P3HA before heating}) \times 100 \ldots (1)$$

(Weight average molecular weight of P3HA before heating)

After 10 mg of P3HA powder had been dissolved in 10 mL of chloroform, insoluble matter was removed by filtration. The weight average molecular weight of this solution (filtrate) was measured using a GPC system manufactured by Shimadzu Corporation equipped with "Shodex K805L (300 mm × 8 mm, two columns connected)" (manufactured by Showa Denko K.K.) with chloroform as a mobile phase. Shodex K-804 (polystyrene gel) manufactured by Showa Denko K.K. was used as a molecular weight standard sample.

(Weight average molecular weight of P3HA after heating)

After the P3HA powder had been preheated at 160°C for 7 minutes, the P3HA powder was heated at 160°C for 20 minutes under a pressure of 5 MPa to prepare a P3HA resin sheet. The weight average molecular weight of P3HA after heating was measured in the same manner as described in (Measurement of weight average molecular weight of P3HA before heating), except that 10 mg of the P3HA resin sheet was dissolved in 10 mL of chloroform.

<Initial molecular weight>

The initial molecular weight was measured by the following procedure. That is, a culture solution containing P3HA after inactivation was diluted with distilled water and centrifuged, and a supernatant was then removed. Ethanol was added to a resulting precipitate (P3HA) so that the precipitate was dispersed in ethanol, and centrifugation was carried out. A supernatant was removed and dried in a vacuum dryer for not less than 1 hour to completely dry a precipitate, so that dry matter (P3HA powder) was obtained. After 10 mg of the obtained P3HA powder had been dissolved in 10 mL of chloroform, insoluble matter was removed by filtration. The initial molecular weight of this solution (filtrate) was measured using a GPC system manufactured by Shimadzu Corporation equipped with "Shodex K805L (300 mm × 8 mm, two columns connected)" (manufactured by Showa Denko K.K.) with chloroform as a mobile phase. Shodex K-804 (polystyrene gel) manufactured by Showa Denko K.K. was used as a molecular weight standard sample.

<Final molecular weight (Mw) and number average molecular weight (Mn)>

The final molecular weight and number average molecular weight were measured by the following procedure. That is, after 10 mg of P3HA powder had been dissolved in 10 mL of chloroform, insoluble matter was removed by filtration. The final molecular weight and number average molecular weight of this solution (filtrate) were measured using a GPC system manufactured by Shimadzu Corporation equipped with "Shodex K805L (300 mm × 8 mm, two columns connected)" (manufactured by Showa Denko K.K.) with chloroform as a mobile phase. Shodex K-804 (polystyrene gel) manufactured by Showa Denko K.K. was used as a molecular weight standard sample.

<Yield of P3HA>

The yield of P3HA was measured by the following procedure:

(a) 2 g of the culture solution before molecular weight adjustment and 2 g of the culture solution after molecular weight adjustment, which will be described later, were taken, and the solid concentration was measured using a moisture meter (ML-50 manufactured by A&D Co., Ltd.).
(b) 5 g of the culture solution before molecular weight adjustment and 5 g of the culture solution after molecular weight adjustment, which will be described later, were weighed into 50 ml falcon tube, and diluted up to 20 ml with ethanol (manufactured by FUJIFILM Wako Pure Chemical Corporation). A slurry was dispersed and then centrifuged (9500 rpm, 5 minutes).
(c) After a supernatant had been removed, distilled water was added to the tube to dilute up to 20 ml and disperse a precipitate, followed by centrifugation (9500 rpm, 5 minutes).
(d) After a supernatant had been removed, a 3.3% aqueous sodium dodecyl sulfate solution (an aqueous solution obtained by dissolving sodium dodecyl sulfate (manufactured by Kao Corporation) in distilled water to a concentration of 3.3% by weight) was added to the tube to dilute up to 30 ml and disperse the precipitate.
(e) Microbial cells were disrupted for 1 minute using an ultrasonic homogenizer (Ultrasonic homogenizer US-150T manufactured by NIHONSEIKI KAISHA LTD.).
(f) The operation (e) was repeated three times.
(g) Centrifugation (9500 rpm, 5 minutes) was carried out. After a supernatant had been removed, distilled water was added to dilute up to 20 ml and disperse a precipitate, followed by centrifugation (9500 rpm, 5 minutes).
(h) After a supernatant had been removed, ethanol was added to the tube to dilute up to 20 ml and disperse a precipitate, followed by centrifugation (9500 rpm, 5 minutes).
(i) A supernatant was removed, followed by drying for 12 hours using a vacuum dryer set at 60°C.
(j) The weight of dry matter obtained in the operation (i) was measured, and the P3HA content and yield were calculated in accordance with the following formulas (3) and (4).

P3HA content (g/g) = (weight of dry matter (g)/weight of culture solution (g)) ... (3)

Yield (%) = (P3HA content of culture solution after molecular weight adjustment/P3HA content of culture solution before molecular weight adjustment) × 100 × (solid concentration (%) in culture solution before molecular weight adjustment/solid concentration (%) in culture solution after molecular weight adjustment) ... (4)

<Residual protein content>

The residual protein content was measured using BCA Protein Assay Kit (manufactured by Thermo Fisher Scientific Inc.).

<SEM>

A carbon tape was applied onto an SEM sample stage. On the carbon tape, P3HA powder containing an external additive or P3HA powder serving as a comparative example was sprinkled, followed by removal of excess powder with a blower. As a pretreatment, coating with a thickness of 20 nm was carried out using an osmium plasma coater. The P3HA powder was pretreated using an osmium plasma coater OPC60A-G manufactured by Filgen Inc. SEM observation was carried out using S-4800 manufactured by Hitachi High-Tech Fielding Corporation.

Example 1

(Preparation of microbial cell culture solution)

*Ralstonia eutropha* described in International Publication No. WO 2019/142717 was cultured by a method described in paragraphs [0041] to [0048] of the same document to obtain a microbial cell culture solution including microbial cells containing P3HA. A composition ratio (a composition ratio of a 3-hydroxybutyrate unit to a 3-hydroxyhexanoate unit) of repeating units of P3HA was 99.9/0.1 (mol/mol) to 96.0/4.0 (mol/mol).

(Inactivation)

The microbial cell culture solution obtained above was subjected to heating and stirring at an internal temperature of 70°C for 8 hours for sterilization.

(Molecular weight adjustment)

The inactivated culture solution (inactivated culture solution) obtained above was adjusted so as to have a pH of 9.0 ± 0.2 with use of 30% sodium hydroxide, the internal temperature was set to 75 ± 2°C, and molecular weight adjustment of P3HA in the microbial cells was carried out for 12 hours. The yield of P3HA was 97% (Table 1).

(Enzyme treatment)

A solution after molecular weight adjustment obtained above (solution after molecular weight adjustment) was adjusted so as to have a pH of 7.0 ± 0.2 by addition of 95% sulfuric acid thereto. After a resulting mixture had been adjusted so as to have a solid concentration of 18% by addition of industrial water (IW) thereto, lysozyme (manufactured by FUJIFILM Wako Pure Chemical Corporation), which is an enzyme (lytic enzyme) that degrades sugar chains (peptidoglycan) in the cell walls, was added, and a resulting mixture was held at 50°C for 2 hours. After that, Alcalase 2.5L (manufactured by Novozyme), which is an alkaline proteolytic enzyme, was added, 30% sodium hydroxide was then added at 50°C, and a resulting mixture was held for 2 hours while being adjusted so as to have a pH of 8.5.

(Lysis and concentration)

30% sodium hydroxide was added to the enzyme-treated solution obtained above to adjust the pH to 8.5 (alkaline cleaning). Then, sodium dodecyl sulfate (SDS, manufactured by Kao Corporation) was added to the enzyme-treated solution so as to be 0.6 wt% to 1.0 wt% (surfactant treatment). After that, the pH was adjusted with use of 30% sodium hydroxide so as to be 11.0 $\pm$ 0.2, and an aqueous sodium hydroxide solution with a pH of 11.0 was added so that the above enzyme-treated solution was diluted by a 2-fold factor.

After the enzyme-treated solution had been centrifuged (4500 rpm, 10 minutes), a supernatant was removed, followed by 2-fold concentration. To a resulting concentrated aqueous suspension of P3HA, an aqueous sodium hydroxide solution (pH 11.0) was added in an amount equal to that of the removed supernatant. A resulting mixture was centrifuged (4500 rpm, 10 minutes), and a supernatant was removed. This process was repeated three times.

Finally, the P3HA concentration was adjusted to 54 $\pm$ 2% by weight by removal of a supernatant, so that an aqueous P3HA suspension was obtained. The residual protein content of the aqueous P3HA suspension was 939 ppm relative to the weight of P3HA present in the P3HA suspension (Table 1).

(Drying)

To the aqueous P3HA suspension obtained above, a nonionic dispersing agent of an ethylene oxide/propylene oxide copolymer (a molecular weight of polyethylene oxide: 8000; a molecular weight of polypropylene oxide: 2000; product name: Plonon 208) was added in an amount of 1.0 phr (1 part by weight relative to 100 parts by weight of P3HA that was present in the aqueous suspension), and a resulting solution was mixed. After this solution had been stirred for 120 minutes, 10 wt% sulfuric acid was added in an amount of 0.3 ml per 100 g of the aqueous P3HA suspension, so that an aqueous P3HA suspension was obtained.

This aqueous P3HA suspension was dried at 60°C for not less than 12 hours to obtain P3HA powder. The obtained P3HA powder had a final molecular weight (Mw) of 173000, a number average molecular weight (Mn) of 70000, and thermal stability of 73% (Table 1). Furthermore, when the state of the surface of the P3HA powder was observed using an electron microscope (SEM; S-4800 manufactured by Hitachi High-Tech Corporation), it was confirmed that the surface of the P3HA powder was a non-porous surface (Fig. 1).

Example 2

A molecular weight-adjusted solution was obtained in the same manner as in Example 1 except for the operations following the operation described under (Molecular weight adjustment).

(Enzyme treatment)

The solution after molecular weight adjustment obtained above (molecular weight-adjusted solution) was adjusted so as to have a solid concentration of 18% by addition of industrial water (IW) thereto. Then, Alcalase 2.5L (manufactured by Novozyme), which is an alkaline proteolytic enzyme, was added, and a resulting mixture was held at 50°C for 2 hours. Then, lysozyme (manufactured by FUJIFILM Wako Pure Chemical Corporation), which is a lytic enzyme that degrades sugar chains (peptidoglycan) in the cell walls, was added, and a resulting mixture was held at 50°C for 2 hours. After that, Alcalase 2.5L (manufactured by Novozyme) was added, and a resulting mixture was held for 2 hours while being adjusted so as to have a pH of 8.5. From this point on, the operation was carried out in the same manner as in Example 1 to obtain P3HA powder. In addition, various measurements were carried out in the same manner as in Example 1.

The yield of P3HA was 97%, and the residual protein content was 760 ppm (Table 1). In addition, the obtained P3HA powder had a final molecular weight (Mw) of 173000, a number average molecular weight (Mn) of 70000, and thermal stability of 70% (Table 1). Furthermore, when the state of the surface of the P3HA powder was observed using an electron microscope (SEM; S-4800 manufactured by Hitachi High-Tech Corporation), it was confirmed that the surface of the P3HA powder was a non-porous surface.

Example 3

P3HA powder was obtained in the same manner as in Example 1 except that P3HA was used in which a composition ratio (a composition ratio of a 3-hydroxybutyrate unit to a 3-hydroxyhexanoate unit) of repeating units of P3HA was 96.0/4.0 (mol/mol) to 92.0/8.0 (mol/mol). In addition, various measurements were carried out in the same manner as in Example 1.

The yield of P3HA was 98%, and the residual protein content was 1647 ppm (Table 1). In addition, the obtained

P3HA powder had a final molecular weight (Mw) of 158000, a number average molecular weight (Mn) of 84000, and thermal stability of 78% (Table 1). Furthermore, when the state of the surface of the P3HA powder was observed using an electron microscope (SEM; S-4800 manufactured by Hitachi High-Tech Corporation), it was confirmed that the surface of the P3HA powder was a non-porous surface.

Example 4

P3HA powder was obtained in the same manner as in Example 1 except that P3HA was used in which a composition ratio (a composition ratio of a 3-hydroxybutyrate unit to a 3-hydroxyhexanoate unit) of repeating units of P3HA was 92.0/8.0 (mol/mol) to 87.0/13.0 (mol/mol). In addition, various measurements were carried out in the same manner as in Example 1.

The yield of P3HA was 99%, and the residual protein content was 1704 ppm (Table 1). In addition, the obtained P3HA powder had a final molecular weight (Mw) of 257000, a number average molecular weight (Mn) of 9 1000, and thermal stability of 80% (Table 1). Furthermore, when the state of the surface of the P3HA powder was observed using an electron microscope (SEM; S-4800 manufactured by Hitachi High-Tech Corporation), it was confirmed that the surface of the P3HA powder was a non-porous surface.

Example 5

An inactivated culture solution was obtained in the same manner as in Example 3 except for the operations following the operation described under (Inactivation).

(Enzyme treatment)

After the inactivated culture solution obtained above had been adjusted so as to have a solid concentration of 18% by addition of industrial water (IW) thereto, lysozyme (manufactured by FUJIFILM Wako Pure Chemical Corporation), which is an enzyme that degrades sugar chains (peptidoglycan) in the cell walls, was added, and a resulting mixture was held at 50°C for 2 hours. After that, Alcalase 2.5L (manufactured by Novozyme), which is a proteolytic enzyme, was added, 30% sodium hydroxide was then added at 50°C, and a resulting mixture was held for 2 hours while being adjusted so as to have a pH of 8.5.

(Molecular weight adjustment)

The enzyme-treated solution obtained above was adjusted so as to have a pH of 11.5 $\pm$ 0.2 with use of 30% sodium hydroxide, the internal temperature was set to 50 $\pm$ 2°C, and molecular weight adjustment of P3HA was carried out for 12 hours. From this point on, the operation was carried out in the same manner as in Example 1 to obtain P3HA particles. In addition, various measurements were carried out in the same manner as in Example 1. Note that the yield of P3HA was measured immediately before the operation described under (Lysis and concentration) (in other words, immediately after the pH was adjusted to 11.5 $\pm$ 0.2, the internal temperature was set to 50 $\pm$ 2°C, and the solution was held for 12 hours).

The yield of P3HA was 30%, and the residual protein content was 796 ppm (Table 1). In addition, the obtained P3HA powder had a final molecular weight (Mw) of 224000, a number average molecular weight (Mn) of 61000, and thermal stability of 3% (Table 1). Furthermore, when the state of the surface of the P3HA powder was observed using an electron microscope (SEM; S-4800 manufactured by Hitachi High-Tech Corporation), it was confirmed that the surface of the P3HA powder was a porous surface.

Example 6

P3HA powder was obtained in the same manner as in Example 5 except that P3HA was used in which a composition ratio (a composition ratio of a 3-hydroxybutyrate unit to a 3-hydroxyhexanoate unit) of repeating units of P3HA was 92.0/8.0 (mol/mol) to 87.0/13.0 (mol/mol). In addition, various measurements were carried out in the same manner as in Example 5.

The yield of P3HA was 96%, and the residual protein content was 1326 ppm (Table 1). In addition, the obtained P3HA powder had a final molecular weight (Mw) of 556000, a number average molecular weight (Mn) of 287000, and thermal stability of 80% (Table 1). Furthermore, when the state of the surface of the P3HA powder was observed using an electron microscope (SEM; S-4800 manufactured by Hitachi High-Tech Corporation), it was confirmed that the surface of the P3HA powder was a non-porous surface.

Comparative Example 1

An enzyme-treated solution was obtained in the same manner as in Example 1 except for the operations following the operation described under (Enzyme treatment).

(Lysis and concentration)

Sodium dodecyl sulfate (SDS, manufactured by Kao Corporation) was added to the enzyme-treated solution obtained above so as to be 0.6 wt% to 1.0 wt%, so that the enzyme-treated solution was diluted by a 2-fold factor. Then, a resulting dilute solution was adjusted so as to have a pH of 8.5 with use of 30% sodium hydroxide. From this point on, the operation was carried out in the same manner as in Example 1 to obtain P3HA particles. In addition, various measurements were carried out in the same manner as in Example 1.

The yield of P3HA was 97%, and the residual protein content was 3547 ppm (Table 1). In addition, the obtained P3HA powder had a final molecular weight (Mw) of 216000, a number average molecular weight (Mn) of 86000, and thermal stability of 63% (Table 1). Furthermore, when the state of the surface of the P3HA powder was observed using an electron microscope (SEM; S-4800 manufactured by Hitachi High-Tech Corporation), it was confirmed that the surface of the P3HA powder was a non-porous surface.

Comparative Example 2

An inactivated culture solution was obtained in the same manner as in Example 1 except for the operations following the operation described under (Inactivation).

(Enzyme treatment)

After the inactivated culture solution obtained above had been adjusted so as to have a solid concentration of 18% by addition of industrial water (IW) thereto, lysozyme (manufactured by FUJIFILM Wako Pure Chemical Corporation), which is an enzyme that degrades sugar chains (peptidoglycan) in the cell walls, was added, and a resulting mixture was held at 50°C for 2 hours. After that, Alcalase 2.5L (manufactured by Novozyme), which is a proteolytic enzyme, was added, 30% sodium hydroxide was then added at 50°C, and a resulting mixture was held for 2 hours while being adjusted so as to have a pH of 8.5.

(Molecular weight adjustment)

The enzyme-treated solution obtained above was adjusted so as to have a pH of 11.5 $\pm$ 0.2 with use of 30% sodium hydroxide, the internal temperature was set to 50 $\pm$ 2°C, and molecular weight adjustment of P3HA was carried out for 12 hours. From this point on, the operation was carried out in the same manner as in Example 1 to obtain P3HA particles. In addition, various measurements were carried out in the same manner as in Example 1. Note that the yield of P3HA was measured immediately before the operation described under (Lysis and concentration) (in other words, immediately after the pH was adjusted to 11.5 $\pm$ 0.2, the internal temperature was set to 50 $\pm$ 2°C, and the solution was held for 12 hours).

The yield of P3HA was 30%, and the residual protein content was 796 ppm (Table 1). In addition, the obtained P3HA powder had a final molecular weight (Mw) of 224000, a number average molecular weight (Mn) of 61000, and thermal stability of 3% (Table 1). Furthermore, when the state of the surface of the P3HA powder was observed using an electron microscope (SEM; S-4800 manufactured by Hitachi High-Tech Corporation), it was confirmed that the surface of the P3HA powder was a porous surface.

Comparative Example 3

P3HA powder was obtained in the same manner as in Comparative Example 2 except that P3HA was used in which a composition ratio (a composition ratio of a 3-hydroxybutyrate unit to a 3-hydroxyhexanoate unit) of repeating units of P3HA was 96.0/4.0 (mol/mol) to 92.0/8.0 (mol/mol). In addition, various measurements were carried out in the same manner as in Example 1.

The yield of P3HA was 85%, and the residual protein content was 1263 ppm (Table 1). In addition, the obtained P3HA powder had a final molecular weight (Mw) of 287000, a number average molecular weight (Mn) of 68000, and thermal stability of 71% (Table 1). Furthermore, when the state of the surface of the P3HA powder was observed using an electron microscope (SEM; S-4800 manufactured by Hitachi High-Tech Corporation), it was confirmed that the surface of the P3HA powder was a porous surface.

Comparative Example 4

P3HA powder was obtained in the same manner as in Comparative Example 2 except that P3HA was used in which a composition ratio (a composition ratio of a 3-hydroxybutyrate unit to a 3-hydroxyhexanoate unit) of repeating units of P3HA was 92.0/8.0 (mol/mol) to 87.0/ 13.0 (mol/mol). In addition, various measurements were carried out in the same manner as in Example 1.

The yield of P3HA was 80%, and the residual protein content was 1806 ppm (Table 1). In addition, the obtained P3HA powder had a final molecular weight (Mw) of 250000, a number average molecular weight (Mn) of 78000, and thermal stability of 57% (Table 1). Furthermore, when the state of the surface of the P3HA powder was observed using an electron microscope (SEM; S-4800 manufactured by Hitachi High-Tech Corporation), it was confirmed that the surface of the P3HA powder was a porous surface.

Comparative Example 5

P3HA powder was obtained in the same manner as in Comparative Example 3, except that the molecular weight adjustment was carried out for 6 hours in the operation described under (Molecular weight adjustment). In addition, various measurements were carried out in the same manner as in Example 1. Note that the yield of P3HA was measured immediately before the operation described under (Lysis and concentration) (in other words, immediately after the pH was adjusted to 11.5 ± 0.2, the internal temperature was set to 50 ± 2°C, and the solution was held for 6 hours).

The yield of P3HA was 94%, and the residual protein content was 1214 ppm (Table 1). In addition, the obtained P3HA powder had a final molecular weight (Mw) of 652000, a number average molecular weight (Mn) of 336000, and thermal stability of 78% (Table 1). Furthermore, when the state of the surface of the P3HA powder was observed using an electron microscope (SEM; S-4800 manufactured by Hitachi High-Tech Corporation), it was confirmed that the surface of the P3HA powder was a non-porous surface.

Comparative Example 6

P3HA powder was obtained in the same manner as in Comparative Example 4, except that the molecular weight adjustment was carried out for 6 hours in the operation described under (Molecular weight adjustment). In addition, various measurements were carried out in the same manner as in Example 1. Note that the yield of P3HA was measured immediately before the operation described under (Lysis and concentration) (in other words, immediately after the pH was adjusted to 11.5 ± 0.2, the internal temperature was set to 50 ± 2°C, and the solution was held for 6 hours).

The yield of P3HA was 88%, and the residual protein content was 1050 ppm (Table 1). In addition, the obtained P3HA powder had a final molecular weight (Mw) of 603000, a number average molecular weight (Mn) of 322000, and thermal stability of 80% (Table 1). Furthermore, when the state of the surface of the P3HA powder was observed using an electron microscope (SEM; S-4800 manufactured by Hitachi High-Tech Corporation), it was confirmed that the surface of the P3HA powder was a non-porous surface.

[Table 1]

| | 3HB/3HH [mol/mol] | Adjustment of molecular weight of microbial cells | Number of alkalase treatments | Number of Alkaline pH adjustments before disruption of microbial cells | Number of Alkaline pH adjustments after disruption of microbial cells | Initial molecular weight [tens of thousands] | Final molecular weight (MN) [tens of thousands] | Number average molecular weight (Mn) [tens of thousands] | Mw/Mn | Particle surface | Residual protein [ppm] | Thermal stability [%] | Yield [%] |
|---|---|---|---|---|---|---|---|---|---|---|---|---|---|
| Example 1 | 99.9/0.1 to 96.0/4.0 | Adjusted | 1 | 1 | 1 | 60 | 17.3 | 7.0 | 2.5 | Non-porous | 939 | 73 | 97 |
| Example 2 | 99.9/0.1 to 96.0/4.0 | Adjusted | 2 | 1 | 1 | 60 | 17.3 | 7.0 | 2.5 | Non-porous | 760 | 70 | 97 |
| Example 3 | 96.014.0 to 92.0/8.0 | Adjusted | 1 | 1 | 1 | 206 | 15.8 | 8.4 | 1.9 | Non-porous | 1647 | 78 | 98 |
| Example 4 | 92.0/8.0 to 87.0/13.0 | Adjusted | 1 | 1 | 1 | 121 | 25.7 | 9.1 | 2.8 | Non-porous | 1704 | 80 | 99 |
| Example 5 | 96.014.0 to 92.0/8.0 | Adjusted | 1 | 1 | 1 | 169 | 56.8 | 29.2 | 1.9 | Non-porous | 1307 | as | 99 |
| Example 6 | 92.018.0 to 87.0/13.0 | Adjusted | 1 | 1 | 1 | 132 | 55.6 | 28.7 | 1.9 | Non-porous | 1326 | 80 | 96 |
| Comparative Example 1 | 99.9/0.1 to 96.0/4.0 | Adjusted | 1 | 1 | 0 | 60 | 21.6 | 8.6 | 2.5 | Non-porous | 3547 | 63 | 97 |
| Comparative Example 2 | 99.9/0.1 to 96.0/4.0 | Not adjusted | 1 | 0 | 1 | 60 | 22.4 | 6.1 | 3.7 | Porous | 796 | 3 | 30 |
| Comparative Example 3 | 96.0/4.0 to 92.0/8.0 | Not adjusted | 1 | 0 | 1 | 165 | 28.7 | 6.8 | 4.2 | Porous | 1263 | 71 | 85 |

(continued)

| | 3HB/3HH [mol/mol] | Adjustment of molecular weight of microbial cells | Number of alkalase treatments | Number of Alkaline pH adjustments before disruption of microbial cells | Number of Alkaline pH adjustments after disruption of microbial cells | Initial molecular weight[tens of thousands] | Final molecular weight(MN) [tens of thousands] | Number average molecular weight (Mn) [tens of thousands] | Mw/Mn | Particle surface | Residual protein [ppm] | Thermal stability [%] | Yield [%] |
|---|---|---|---|---|---|---|---|---|---|---|---|---|---|
| Comparative Example 4 | 92.018.0 to 87.0/13.0 | Not adjusted | 1 | 0 | 1 | 121 | 25.0 | 7.8 | 3.2 | Porous | 1806 | 57 | 80 |
| Comparative Example 5 | 96.014.0 to 92.0/8.0 | Not adjusted | 1 | 0 | 1 | 169 | 65.2 | 33.6 | 1.9 | Non-porous | 1214 | 78 | 94 |
| Comparative Example 6 | 92.018.0 to 87.0/13.0 | Not adjusted | 1 | 0 | 1 | 132 | 60.3 | 32.2 | 1.9 | Non-porous | 1050 | 80 | 88 |

Results

Table 1 shows physical properties measured in Examples 1 to 6 and Comparative Examples 1 to 6, and Fig. 3 shows the operations performed in Examples 1 to 6 and Comparative Examples 1 to 6 and the order of the operations.

In Table 1, a comparison between Examples 1 and 2 and Comparative Example 1 shows that holding under alkaline conditions after enzyme treatment (that is, outside the microbial cells) enables the residual protein content to be significantly reduced. Further, a comparison between Examples 1 to 6 and Comparative Examples 2 to 6 shows that a high yield of P3HA is achieved by adjusting the molecular weight of P3HA before enzyme treatment (that is, inside the microbial cells). Further, a comparison between Examples 1 to 4 and Comparative Examples 2 to 4 shows that, when molecular weight adjustment is carried out to lower the molecular weight to 150,000 to 300,000, another molecular weight adjustment carried out after enzyme treatment (that is, outside the microbial cells) enables the surface of P3HA powder to be non-porous.

Industrial Applicability

According to the present invention, it is possible to produce, at a high yield, P3HA in which impurities (in particular, residual protein) are reduced. Further, the P3HA in which impurities (in particular, residual protein) are reduced and which has been obtained by a production method of the present invention can be suitably used in the fields of agriculture, fishery, forestry, horticulture, medicine, hygiene, clothing, non-clothing, packaging, motor vehicles, and building materials, and other fields.

## Claims

1. A method for producing poly(3-hydroxyalkanoate) having a weight average molecular weight of 100,000 to 700,000, the method comprising:

   (a) a step of adding an aqueous alkaline solution to a culture solution including microbial cells containing poly(3-hydroxyalkanoate) to adjust the pH of the culture solution to 8.0 to 12.0;
   (b) a step of adding an enzyme to the culture solution obtained in the step (a) to subject the microbial cells to an enzyme treatment; and
   (c) a step of adding an aqueous alkaline solution to the culture solution obtained in the step (b) to adjust the pH of the culture solution to 10.0 to 12.0.

2. The method according to claim 1, wherein the enzyme treatment in the step (b) comprises carrying out an alkaline proteolytic enzyme treatment, a lytic enzyme treatment, and another alkaline proteolytic enzyme treatment in this order.

3. The method according to claim 1 or 2, further comprising, between the steps (a) and (b), (a') a step of adjusting the pH of the culture solution obtained in the step (a) to 6.0 to 8.0, wherein
   the enzyme treatment in the step (b) comprises carrying out a lytic enzyme treatment and an alkaline proteolytic enzyme treatment in this order.

4. The method according to any one of claims 1 to 3, wherein the enzymes in the step (b) are lysozyme and alcalase.

5. The method according to any one of claims 1 to 4, further comprising, after the step (c), (c') a step of adding a surfactant to the culture solution obtained in the step (c).

6. The method according to any one of claims 1 to 5, wherein a reaction time in the step (a) is 4 hours to 30 hours.

7. The method according to any one of claims 1 to 6, wherein the poly(3-hydroxyalkanoate) is poly(3-hydroxybutyrate-co-3-hydroxyhexanoate).

8. Poly(3-hydroxyalkanoate) powder having a weight average molecular weight of 100,000 to 600,000, having a residual protein content of not more than 2000 ppm, having a particle surface which is non-porous, and having a weight average molecular weight retention rate of not less than 50%, the weight average molecular weight retention rate represented by the following formula (1):
   Weight average molecular weight retention rate (%) = (weight average molecular weight of P3HA after heating at

160°C for 20 minutes/weight average molecular weight of P3HA before heating at 160°C for 20 minutes) × 100 ... (1)

**9.** The poly(3-hydroxyalkanoate) powder according to claim 8, wherein a ratio (Mw/Mn) of a weight average molecular weight (Mw) of the poly(3-hydroxyalkanoate) powder to a number average molecular weight (Mn) thereof is 1.0 to 3.1.

FIG. 1

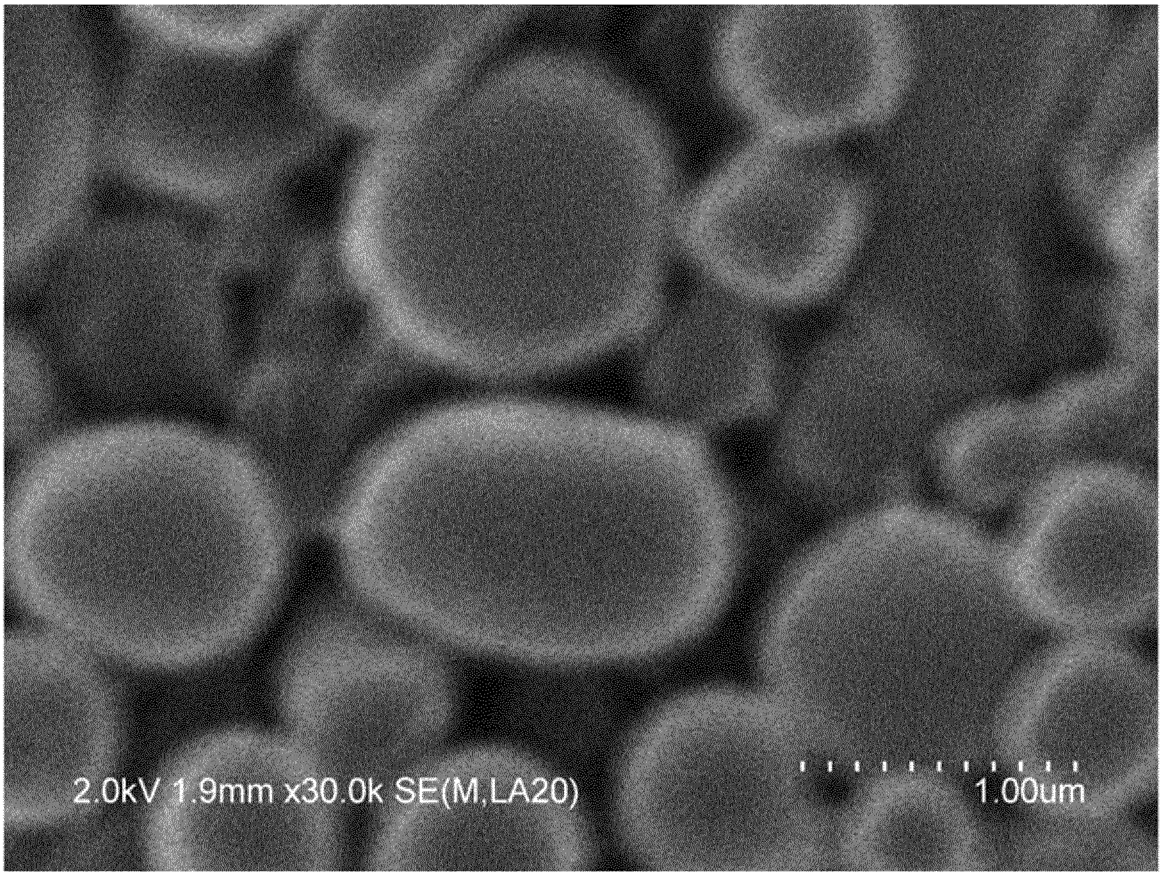

FIG. 2

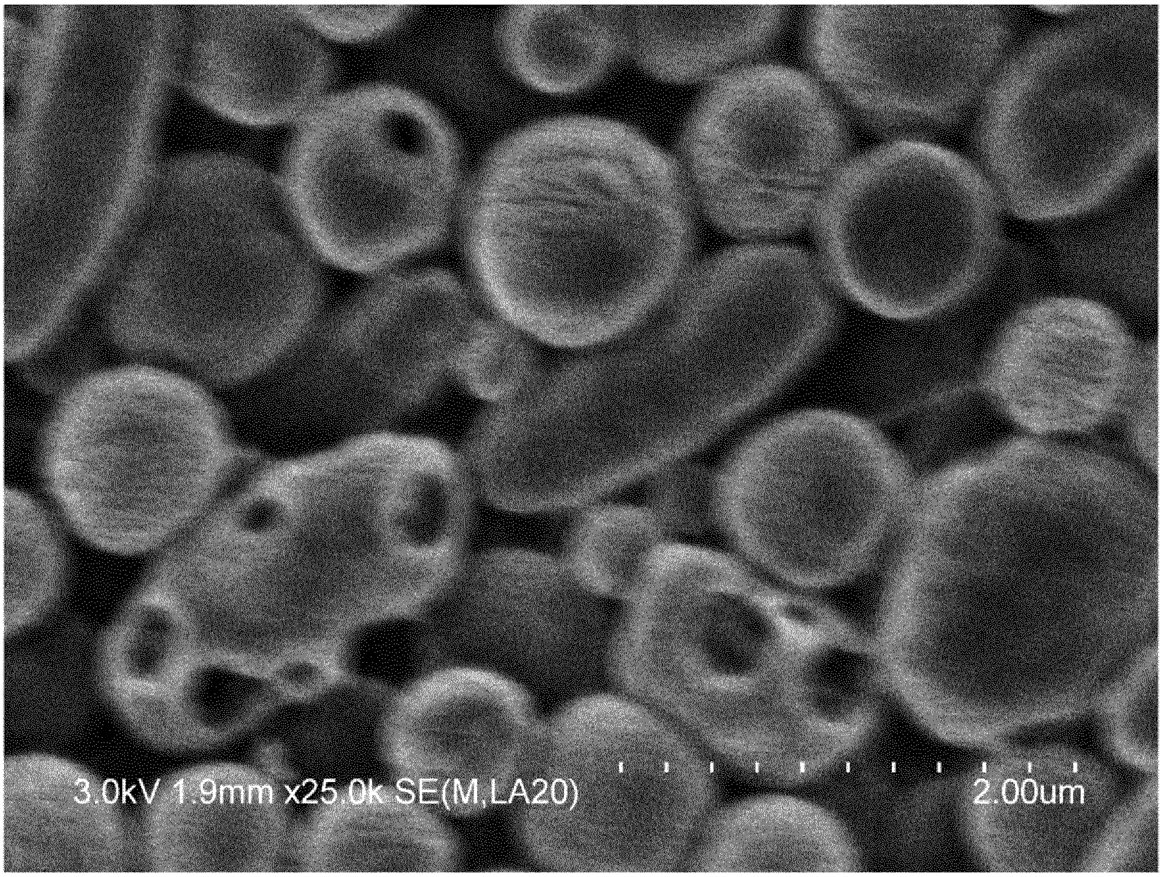

FIG. 3

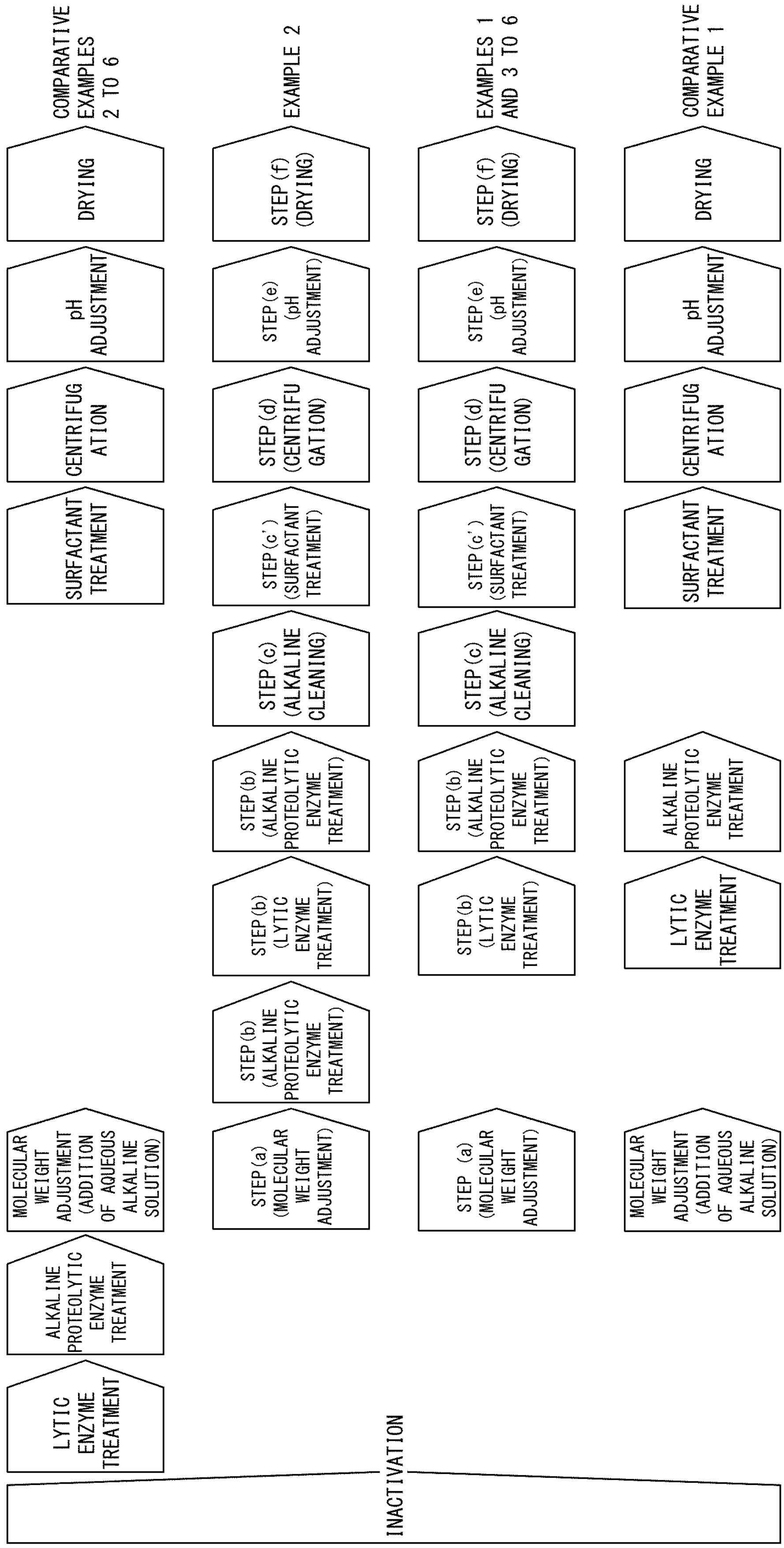
INACTIVATION
LYTIC ENZYME TREATMENT
ALKALINE PROTEOLYTIC ENZYME TREATMENT
MOLECULAR WEIGHT ADJUSTMENT (ADDITION OF AQUEOUS ALKALINE SOLUTION)
SURFACTANT TREATMENT
CENTRIFUGATION
pH ADJUSTMENT
DRYING
COMPARATIVE EXAMPLES 2 TO 6
STEP (a) (MOLECULAR WEIGHT ADJUSTMENT)
STEP (b) (ALKALINE PROTEOLYTIC ENZYME TREATMENT)
STEP (b) (LYTIC ENZYME TREATMENT)
STEP (b) (ALKALINE PROTEOLYTIC ENZYME TREATMENT)
STEP (c) (ALKALINE CLEANING)
STEP (c') (SURFACTANT TREATMENT)
STEP (d) (CENTRIFUGATION)
STEP (e) (pH ADJUSTMENT)
STEP (f) (DRYING)
EXAMPLE 2
STEP (a) (MOLECULAR WEIGHT ADJUSTMENT)
STEP (b) (LYTIC ENZYME TREATMENT)
STEP (b) (ALKALINE PROTEOLYTIC ENZYME TREATMENT)
STEP (c) (ALKALINE CLEANING)
STEP (c') (SURFACTANT TREATMENT)
STEP (d) (CENTRIFUGATION)
STEP (e) (pH ADJUSTMENT)
STEP (f) (DRYING)
EXAMPLES 1 AND 3 TO 6
MOLECULAR WEIGHT ADJUSTMENT (ADDITION OF AQUEOUS ALKALINE SOLUTION)
LYTIC ENZYME TREATMENT
ALKALINE PROTEOLYTIC ENZYME TREATMENT
SURFACTANT TREATMENT
CENTRIFUGATION
pH ADJUSTMENT
DRYING
COMPARATIVE EXAMPLE 1

**INTERNATIONAL SEARCH REPORT**

International application No.
**PCT/JP2021/036938**

**A. CLASSIFICATION OF SUBJECT MATTER**

***C12P 7/62***(2022.01)i; ***C08G 63/89***(2006.01)i; ***C12P 7/42***(2006.01)i
FI: C12P7/62; C12P7/42; C08G63/89

According to International Patent Classification (IPC) or to both national classification and IPC

**B. FIELDS SEARCHED**

Minimum documentation searched (classification system followed by classification symbols)

C12P7/62; C08G63/89; C12P7/42

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | WO 2010/116681 A1 (KANEKA CORP.) 14 October 2010 (2010-10-14)<br>claims, paragraphs [0023], [0024], [0026], [0032]-[0036], [0053], examples, table 1 | 1-9 |
| A | WO 2005/049692 A1 (KANEKA CORP., THE PROCTER & GAMBLE CO.) 02 June 2005 (2005-06-02)<br>claims, paragraph [0019], examples | 1-9 |
| A | WO 2004/065608 A1 (KANEKA CORP.) 05 August 2004 (2004-08-05)<br>claims, page 6, line 43 to page 7, line 18, page 10, line 18 to page 12, line 40, examples | 1-9 |
| A | WO 2005/085461 A1 (KANEKA CORP.) 15 September 2005 (2005-09-15)<br>claims, paragraphs [0022]-[0025], [0032], [0033], [0059], examples | 1-9 |
| A | JP 2007-524345 A (TIANAN BIOLOGIC MATERIAL CO., LTD.) 30 August 2007 (2007-08-30)<br>claims, paragraph [0004], examples | 1-9 |
| A | WO 2020/218565 A1 (FUENCE CO., LTD.) 29 October 2020 (2020-10-29)<br>claims, paragraphs [0022], [0033]-[0037], [0057], examples | 1-9 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 November 2021** | **30 November 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

INTERNATIONAL SEARCH REPORT

International application No. **PCT/JP2021/036938**

**C. DOCUMENTS CONSIDERED TO BE RELEVANT**

| Category* | Citation of document, with indication, where appropriate, of the relevant passages | Relevant to claim No. |
|---|---|---|
| A | JIANG, Yang et al. Feasibility study of an alkaline-based chemical treatment for the purification of polyhydroxybutyrate produced by a mixed enriched culture. AMB Express, 2015, 5: 5 pages 1-13 | 1-9 |

Form PCT/ISA/210 (second sheet) (January 2015)

**INTERNATIONAL SEARCH REPORT**
**Information on patent family members**

International application No.
**PCT/JP2021/036938**

| Patent document cited in search report | Publication date (day/month/year) | Patent family member(s) | Publication date (day/month/year) |
|---|---|---|---|
| WO 2010/116681 A1 | 14 October 2010 | (Family: none) | |
| WO 2005/049692 A1 | 02 June 2005 | EP 001693397 A1<br>claims, paragraph [0031], examples<br>US 2005/0222373 A1 | |
| WO 2004/065608 A1 | 05 August 2004 | EP 001609868 A1<br>claims, paragraphs [0021]-[0025], [0052]-[0084], examples<br>US 2006/0084161 A1 | |
| WO 2005/085461 A1 | 15 September 2005 | US 2005/0196827 A1<br>claims, paragraphs [0042]-[0046], [0042], [0057], [0093], examples | |
| JP 2007-524345 A | 30 August 2007 | EP 001705250 A1<br>claims, paragraph [0003], examples<br>US 2007/0072276 A1<br>WO 2005/059153 A1 | |
| WO 2020/218565 A1 | 29 October 2020 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2012115145 A
- CN 111019108
- JP 5093049 A
- WO 2019142717 A